# EUROPEAN PATENT APPLICATION

(11) **EP 1 854 806 A1**
(43) Date of publication of application: **14.11.2007**
(21) Application number: 06009058.6
(22) Date of filing: 02.05.2006
(51) Int. Cl.: C07H 15/203

(54) **Thioglycosides as pharmaceutically active agents**

(71) Applicant: MPG Max-Planck-Gesellschaft zur Förderung der Wissenschaften e.V., 80539 München (DE)
(72) Inventor: Castaneda, Francisco, 44388 Dortmund (DE); Kinne, Rolf K-H., 44227 Dortmund (DE); Boland, Wilhelm, 07743 Jena (DE); Burse, Antje, 07749 Jena (DE)
(74) Representative: Arth, Hans-Lothar

(57) **Abstract**

The present invention relates to thioglycosides and stereoisomeric forms, solvates, hydrates and/or pharmaceutically acceptable salts of these compounds, as well as pharmaceutical compositions containing at least one of these thioglycosides together with pharmaceutically acceptable carrier, excipient and/or diluents. Said thioglycosides have been identified as specific inhibitors of the sodium dependent glucose transporter 1 (SGLT1) and 2 (SGLT2), and are useful for the prophylaxis and/or treatment of hyperglycemia and hyperglycemia related diseases and conditions.

## Description

The present invention relates to thioglycosides and stereoisomeric forms, solvates, hydrates and/or pharmaceutically acceptable salts of these compounds, as well as pharmaceutical compositions containing at least one of these thioglycosides together with pharmaceutically acceptable carrier, excipient and/or diluents. Said thioglycosides have been identified as specific inhibitors of the sodium dependent glucose transporter 1 (SGLT1) and 2 (SGLT2), respectively, and are useful for the prophylaxis and/or treatment of hyperglycemia and hyperglycemia related diseases and conditions.

### Background of the invention

The transport of glucose into epithelial cells is regulated by a secondary active transport system, the sodium-dependent D-glucose transporters (SGLT1 and SGLT2), driven by a sodium-gradient formed by Na⁺/K⁺-ATPase. Glucose accumulated in the epithelial cell is further transported into the blood across the membrane by facilitated diffusion through GLUT transporters. In the kidney, glucose is filtered in the glomerulus and reabsorbed into the renal proximal tubular epithelial cells by SGLT2 in the S1 and S2 tubular segments. Much smaller amounts of glucose are recovered by SGLT1 in the later segment of the tubule. In the small intestine SGLT1 is the transporter mainly involved in sugar absorption.

Considering the physiologic effect of glucose reabsorption in the kidney and absorption in the intestine, they represent important therapeutic targets for the regulation of blood glucose concentration, thus, controlling hyperglycemia.

The role of glucose analogues in SGLT inhibition has been well demonstrated in *in vitro* animal models (Oku et al., Diabetes 48(9), 1794 -1800 (1999); Ohsumi et al., Bioorganic & Medicinal Chemistry Letters 13(14), 2269-2272 (2003)) and *in vivo* animal models (Oku et al., Biological and Pharmaceutical Bulletin 23(12), 1434-1437 (2000); Ueta et al., Life Science 76(23), 2655-68 (2005)). The most studied substance is phlorizin (Ehrenkranz et al., Diabetes Metab Res Rev 21(1), 31-8 (2005)) an O-glucoside derivative. The clinical application of O-glucoside derivatives, however, is restricted due to hydrolysis by β-glucosidases in the intestine. To overcome this problem, phlorizin analogues such as T-1095 (3-(benzofuran-5-yl)-2',6' -dihydroxy -4'- methylpropiophenone 2'-O- (6-O-methoxycarbonyl -β-D-glyco-pyrano-side) have been chemically synthesized. T-1095 is absorbed through the small intestine and is then converted into its active form, resulting in the inhibition of glucose reabsorption in the renal tubules (Ohsumi et al., Bioorganic & Medicinal Chemistry Letters 13(14); Nunoi et al., Clinical and Experimental Pharmacology and Physiology 29(5-6), 386-90 (2002)).

The use of O-glucoside derivatives for the inhibition of SGLT2 has also been described, for example, in WO 01/74834 A1, WO 03/020737 A1, WO 2005/011592 A2, WO 2005/012242 A2 and WO 2005/012243 A2 and the use of C-glucoside derivatives for the inhibition of SGLT2 has been described, for example, in WO 01/27128 A1 and WO 02/083066 A2.

Herein, we report the discovery of thioglycoside derivatives which demonstrate selective inhibition of human SGLT1 and SGLT2, respectively.

The production of thioglycoside derivatives has been described, for example, in CH 539071A, WO 2004/024908 A1 and WO 2005/040371 A1.

Thioglycoside derivatives have been described for use in the prevention of bacterial plaque formation in JP 07126132 A and for use as a growth promoter for animals and microorganisms in JP 62026204 A.

JP 2002069000 A describes the use of a thioglycoside derivative as a liposome surfactant for the production of an insulin containing liposome suitable for oral administration.

None of these prior art documents disclose the use of the claimed thioglycoside derived compounds described in the present application for the inhibition of SGLT 1 and/or SGLT2 for the prophylaxis and/or treatment of hyperglycemia or hyperglycemia related diseases and conditions.

It is the object of the present invention to provide compounds and/or pharmaceutically acceptable salts thereof which can be used as pharmaceutically active agents, especially for prophylaxis and/or treatment of hyperglycemia and hyperglycemia related diseases and conditions, as well as compositions comprising at least one of those compounds and/or pharmaceutically acceptable salts thereof as pharmaceutically active ingredients.

The object of the present invention is solved by the teaching of the independent claims. Further advantageous features, aspects and details of the invention are evident from the dependent claims, the description, and the examples of the present application.

### Description of the invention

The thioglycoside derivatives according to the present invention are represented by the following general formula (I) 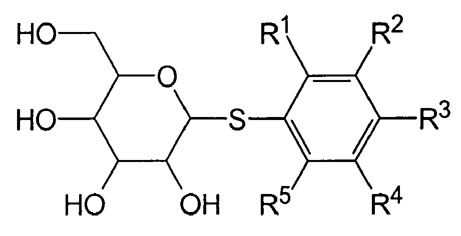 wherein
R¹ - R⁵ represent independently of each other
   -R⁶, -R⁷, -R⁸, -R⁹, -R¹⁰, -NHR¹¹, -NHR¹², -NR¹³R¹⁴, -NR¹⁵R¹⁶, -OR¹⁷, -OR¹⁸, -COR¹⁹, -COR²⁰, -CONH-R²¹, -CONH-R²², -COOR²³, -COOR²⁴, -OOC-R²⁵, -OOC-R²⁶, -NHCO-R²⁷, -NHCO-R²⁸, -R²⁹, -R³⁰, -R³¹ -R³², -R³³,
R⁶ - R²⁸ represent independently of each other
   -R³⁴, -R³⁵, -R³⁶, -R³⁷, -R³⁸, -CH₂-R³⁹, -C₂H₄-R⁴⁰, -C₃H₆-R⁴¹, -C₄H₈-R⁴². -C₅H₁₀-R⁴³,-C₆H₁₂-R⁴⁴, -C₇H₁₄-R⁴⁵, -CHR⁴⁶R⁴⁷, -CR⁴⁸R⁴⁹R⁵⁰, -CHR⁵¹-CHR⁵²R⁵³, -CR⁵⁴R⁵⁵-CR⁵⁶R⁵⁷R⁵⁸, -CHR⁵⁹-CHR⁶⁰-CHR⁶¹R⁶², -CR⁶³R⁶⁴-CR⁶⁵R⁶⁶-CR⁶⁷R⁶⁸R⁶⁹; 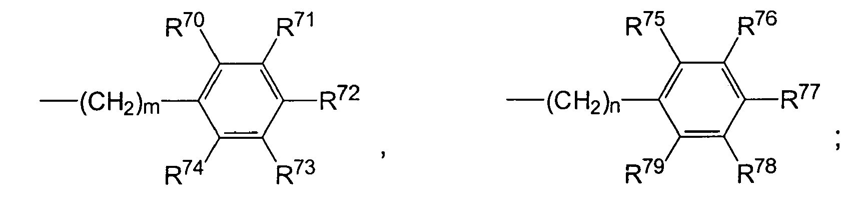
R²⁹ - R⁷⁹ represent independently of each other
   -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -l, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-CH₃, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃. -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CHr-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH2-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂. -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂. -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂. -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C=C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH. -CHr-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃;
m is the integer 1, 2, 3, 4, 5, 6, 7 or 8;
n is the integer 1, 2, 3, 4, 5, 6, 7, 8, 9 or 10;
and wherein at least one substituent R¹ - R⁵ is different from hydrogen, and stereoisomeric forms, enantiomers, diastereomers, tautomers, anomers, keto-forms, deoxy-forms, prodrugs, solvates, hydrates and pharmaceutically acceptable salts thereof.

Preferred are these compounds wherein a substituent in ortho position is present. Also preferred are compounds wherein a substituent in meta position is present. Still preferred are compounds wherein two or three or four substituents in ortho and/or meta position are present.

The glycose moiety (the term "glycose" refers to any six carbon containing sugar) in the above general formula (I) can be any of the following six carbon glycoses:

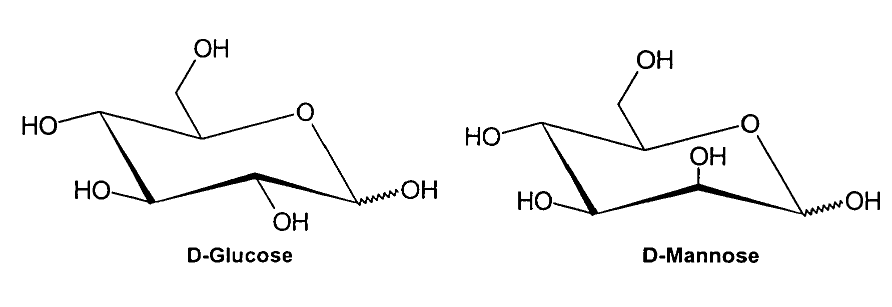

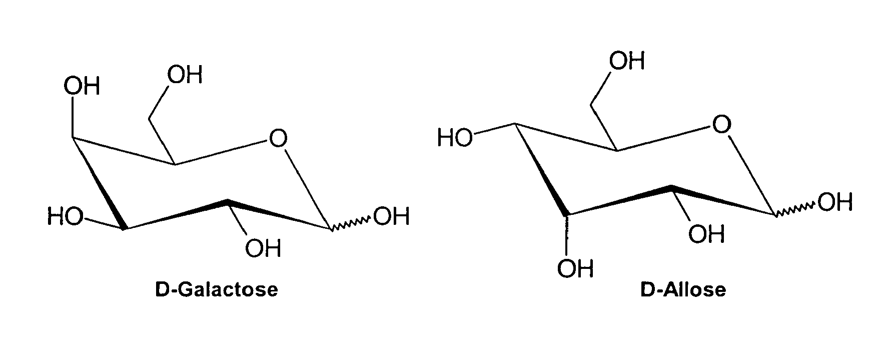

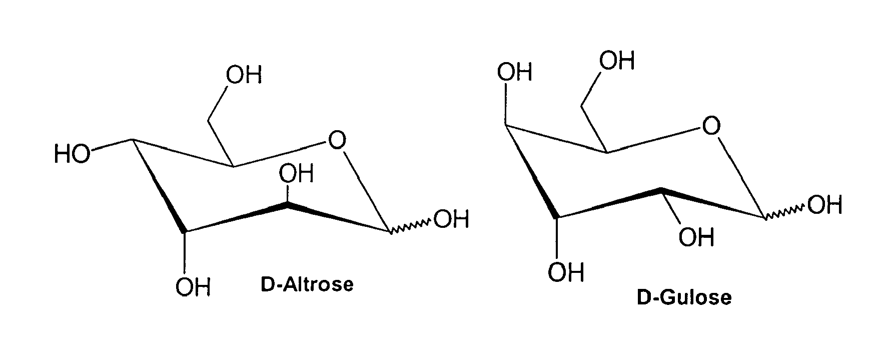

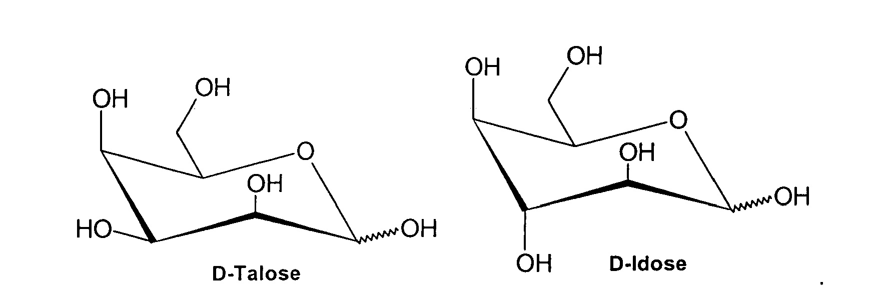

Preferred are the thioglycoside derivatives wherein the glycoside is glucose as shown in the following general formula (II):

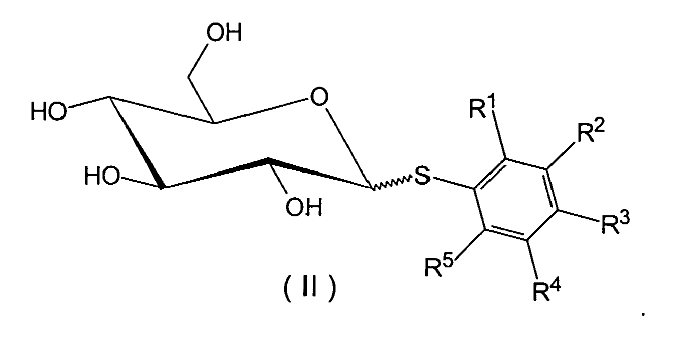

Compounds wherein the -S-phenyl residue is below the plane of the sugar ring are called α-anomers or α-glycosides and wherein the -S-phenyl residue is above the plane of the sugar ring are called β-anomers or β-glycosides. Preferred are the compounds wherein the -S-phenyl residue is in equatorial position.

Also preferred are the thioglycoside derivatives wherein the glycoside is galactose as shown in the following general formula (III):

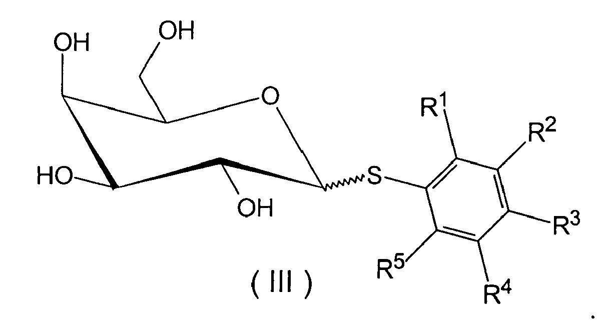

Also preferred are the thioglycoside derivatives wherein R¹ and/or R⁵ represents -CH₂OH, -CH₂-CH₂OH, -CH₂-CH₂NH₂, -CH₂-CH₂SH, -CH₂NH₂, -CH₂SH and most preferred -CH₂OH.

The above-mentioned thioglycoside derivatives are especially useful as pharmaceutically active agents, most especially for prophylaxis and/or treatment of hyperglycemia and hyperglycemia related diseases and conditions.

The present invention also comprises pharmaceutically acceptable salts of the compounds according to the general formula (I), all stereoisomeric forms of the compounds according to the general formula (I) as well as solvates, especially hydrates thereof.

In the case the inventive compounds bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner. In case, the compounds bear acidic substituents, the formation of salts with inorganic or organic bases may be possible. Examples for such bases are NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallized or recrystallized from solvents such as aqueous and organic solvents. In such cases solvates may be formed. This invention includes within its scope stoichiometric solvates including hydrates as well as compounds containing variable amounts of water that may be produced by processes such as lyophilization.

Some compounds of the general formula (I) may exist in the form of optical isomers, e.g. enantiomers, diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomer(s).

Another aspect of the present invention relates to the use of the inventive thioglycoside derivatives as drugs, i.e. as pharmaceutically active agents applicable in medicine.

Inhibition of human sodium dependent glucose transporter 2 (SGLT2) mediated glucose reabsorption in the kidney represents a promising therapeutic target. SGLT2 is responsible for most of the reabsorption of glucose in the kidney.

It was found that the thioglycoside derivatives of the present invention are specific inhibitors of human SGLT2. Inhibitors of SGLT2 or the inhibition of SGLT2 cause the inhibition of glucose reabsorption in the kidney.

Thus, the thioglycoside derivatives disclosed herein are especially useful for the manufacture of a pharmaceutical compositions useful as inhibitors of the human SGLT2.

Further, the thioglycoside derivatives disclosed herein are especially useful for the prophylaxis and/or treatment of diseases and conditions associated with glucose transport by SGLT2.

Inhibition of glucose transport in the kidney represents a different mechanism of action from the other agents commonly used in clinical practice to decrease blood glucose concentration (anti-hyperglycemic agents). Inhibition of glucose transport in the kidney leads to increased urinary glucose excretion and consequently decreases blood glucose concentration. The decrease of glucose transport induced by thioglucosides leads to reduction of blood glucose concentration with subsequently decrease of glomerular filtration. Thus, the effect of these thioglucosides in the kidney is auto-regulated by the blood glucose concentration avoiding a significant reduction of blood glucose concentration, which represents a common complication observed when using other anti-hyperglycemic agents.

Diabetes mellitus is characterized by reduced insulin secretion from pancreatic b-cells (type 1 diabetes) or deficient insulin action (type 2 diabetes), both causing an increase in the blood glucose concentration (hyperglycemia). Hyperglycemia represents the main pathogenic factor for the development of diabetic complications including coronary heart disease, retinopathy, nephropathy, and neuropathy.

In addition, chronic hyperglycemia leads to progressive impairment of insulin secretion and to insulin resistance of peripheral tissues (referred to as glucose toxicity).

Thus, the thioglycoside derivatives disclosed herein are especially useful for the treatment of hyperglycemic diseases and conditions such as type I diabetes, type II diabetes and diabetic complications including coronary heart disease, retinopathy, nephropathy, neuropathy and delayed wound healing.

As further hyperglycemic diseases and conditions the following can also be mentioned: hyperinsulinemia; insulin resistance; obesity; hyperlipidemia including hypertriglyceridemia; metabolic syndrome X, also called insulin resistance syndrome; atherosclerosis; hypertension and related diseases.

Preferred as SGLT2 inhibitors and preferred for prophylaxis and/or treatment of hyperglycemic diseases and conditions are the compounds of general formula (I) wherein the glycose moiety is glucose. Also preferred are the compounds of general formula (I) wherein the glycose moiety is galactose.

Preferred are also the compounds of general formula (I) wherein R¹ or R⁵ represents -CH₂OH.

In further preferred embodiments of the present invention, at least one compound of general formula (I) - (III) is applied in combination with existing anti-hyperglycemic agents for the treatment or prophylaxis of type I diabetes, type II diabetes, or diabetic complications including coronary heart disease, retinopathy, nephropathy, neuropathy, delayed wound healing, hyperinsulinemia; insulin resistance; obesity; hyperlipidemia including hypertriglyceridemia; metabolic syndrome X, also called insulin resistance syndrome; atherosclerosis; hypertension and related diseases.

The mechanisms of action of existing anti-hyperglycemic agents have been focused on improved glucose disposal, controlled hepatic glucose release or inhibition of intestinal glucose absorption.

It is preferred to use at least one compound of general formula (I) - (III) in combination with an anti-diabetic or anti-hyperglycemic agent such as insulin and insulin analogues, insulin sensitizers, meglitinides, bigaunides, sulfonyl ureas, PPAR inhibitors, glucagon-like peptide-1 (GLP-1), PTP1 B inhibitors, glycogen phosphorylase inhibitors and glucose-6-phosphatase inhibitors.

It is also preferred to use at least one compound of general formula (I) - (III) in combination with agents for treating diabetic complications such as PKC inhibitors or AGE inhibitors.

It is also preferred to use at least one compound of general formula (I) - (III) in combination with a lipid-lowering agent such as HMG CoA reductase inhibitors, MTP inhibitors, squalene synthetase inhibitors, fibric acid derivatives, ACAT inhibitors, lipooxygenase inhibitors, cholesterol absorption inhibitors, ileal Na+/bile acid cotransporter inhibitors, upregulators of LDL receptor activity, bile acid sequestrants, and nicotinic acid

It is further preferred to use at least one compound of general formula (I) - (III) in combination with an anti-obesity agent such as a beta 3 adrenergic agonist, a lipase inhibitor, a serotonin reuptake inhibitor, and thyroid receptor beta drug.

Still another aspect of the present invention relates to pharmaceutical compositions comprising at least one compound according to general formula (I) - (III) as an active ingredient, together with at least one pharmaceutically acceptable carrier, excipient, binders, disintegrates, glidents, diluents, lubricants, coloring agents, sweetening agents, flavoring agents, preservatives or the like. The pharmaceutical compositions of the present invention can be prepared in a conventional solid or liquid carrier or diluents and a conventional pharmaceutically-made adjuvant at suitable dosage level in a known way.

The preferred preparations are adapted for oral application. These administration forms include, for example, pills, tablets, film tablets, coated tablets, capsules, liposomal formulations, micro- and nano-formulations, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient. Preparations for oral use are preferred.

The pharmaceutical compositions according to the present invention containing at least one compound according to the present invention and/or a pharmaceutically acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. The pharmaceutical formulation may further contain a cytotoxic and/or cytostatic drug. Other suitable formulations are gels, elixirs, dispersible granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

As pharmaceutically acceptable carrier, excipient and/or diluents can be used lactose, starch, sucrose, cellulose, magnesium stearate, dicalcium phosphate, calcium sulfate, talc, mannitol, ethyl alcohol (liquid filled capsules).

Suitable binders include starch, gelatine, corn sweeteners, natural and synthetic gums such as acacia, sodium alginate, carboxymethylcellulose, polyethylene glycol and waxes, sugars such as sucrose, starches derived from wheat corn rice and potato, natural gums such as acacia, tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate.

Lubricants such as boric acid, sodium benzoate, sodium acetate, sodium chloride, magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine may be used within the pharmaceutical preparations.

Disintegrating agents (disintegrates) such as starch, methylcellulose, guar gum, modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures can be used.

Furthermore, coloring agents, sweetening agents, flavoring agents and/or preservatives may be present in the pharmaceutical formulations.

Glidents which can be used are for example silicon dioxide and talc; suitable adsorbent are clay, aluminum oxide.

Suitable diluents are water or water/propylene glycol solutions for parenteral injections, juice, sugars such as lactose, sucrose, mannitol, and sorbitol, starches derived from wheat, corn rice, and potato, and celluloses such as microcrystalline cellulose.

Said pharmaceutical compositions may further comprise at least one of the above-mentioned anti-hyperglycemic agents, anti-diabetic agents, agents for treating diabetic complications, lipid-lowering agents or anti-obesity agents.

### Description of the figures:

- Figure 1: Determination of SGLT translocation activity assessed using fluorescence resonance energy transfer (FRET). FRET signal obtained by each thioglycoside, D-glucose and phlorizin on human SGLT1 and SGLT2 expressed as relative fluorescence units.
- Figure 2: [¹⁴C]AMG-uptake obtained in human SGLT1 or SGLT2 treated with thioglycosides 1 through 8 or phlorizin (10 µM each). Results represent the mean of 16 determinations (four experiments) and are expressed as % of inhibition.

### EXAMPLES

### Materials and Methods

### Cell Culture

Stable transfected Chinese hamster ovary (CHO) cells that express human SGLT1 or human SGLT2 were seeded at a concentration of 1 x 10³ cells/ml and maintained in culture for 2 days to allow the cells to form a confluent monolayer culture. For transport studies cells were seeded in 96-well microtiter scintiplates (PerkinElmer, Weisbaden, German). For fluorescence resonance energy transfer (FRET) analysis cells were seeded in flat-bottom, poly-D-lysine black-wall, clear bottom, 96-well plates (Becton Dickinson; Heidelberg, Germany).

### SGLT Activity

SGLT activity, as indicated by membrane translocation, was assessed using fluorescence resonance energy transfer (FRET). Cells were incubated for 48 hours ar 37°C in a 5% CO₂ atmosphere in growth medium. Cells were washed with 0.2 ml Dulbecco's phosphate-buffered saline (Invitrogen, Karlsruhe, Germany) and then incubated with 0.1 ml of a solution containing 5 µm CC2-DMPE and 0.02% pluronic acid in PBS. After incubation in the dark for 30 min at 25°C, cells were washed twice with 0.2 ml PBS. Afterwards, cells were incubated in the dark at 25°C for 30 minutes with 0.1 ml of a solution containing 1 Mm DiSBAC₂(3). A t the end of the incubation period 1 mM of each thioglucoside was added and excited by 390 nm. Fluorescence emission was recorded at 460 and 580 nm. After a 20 second baseline reading, 0.1 ml of PBS containing 10 µM of each compound was added, and the fluorescence was calculated as the ratio F/F₀ = [(A₄₆₀/A₅₈₀)/(I₄₆₀/I₅₈₀)] where A and I represent the reading after or before addition of each thioglucoside, respectively. For I, the readings form 2-5 seconds were averaged; and for A, readings from 3 seconds after the signal that had reached a plateau level (usually within 2-5 seconds) were also averaged. FRET values were expressed as relative fluorescence units (RFU).

### Inhibition of SGLT Transport

Sodium-dependent D-glucose transport was determined by means of α-methyl [¹⁴C]D-glucopyranoside ([¹⁴C]AMG, spec. Radioactivity 300 mCi/mmol) purchased from NEN (Bad Homburg, Germany). For the purpose of this study, Krebs-Ringer-Henseleit (KRH) solution containing 120 mM NaCl, 4.7 mM KCI, 1.2 mM MgCl₂, 2.2 mM CaCl₂, 10 mM Hepes (pH 7.4 with Tris) was used to assess sodium-dependent D-glucose transport. For sodium free conditions, KRH solution containing 120 mM N-methyl-glucamine (NMG) instead of NaCl (Na⁺) was used to assess sodium-independent D-glucose transport. All chemicals were purchased from Sigma (Deisenhof, Germany).

Briefly, cells were rinsed three times with 200 µl KRH-Na⁺ or KRH-NMG. Then, 100 µl per well of transport buffer containing KRH-NA⁺ or KRH-NMG plus [¹⁴C]AMG (0.1 µCi/µl) were added and incubated for 1 hour. At the end of the uptake period, [¹⁴ C]AMG-uptake was stopped by adding 100 µl of ice-cold stop buffer (KRH-Na⁺, containing 0.5 mM phlorizin). Then, the cells were solubilized by adding 100 µl of ATPlite substrate solution (PerkinElmer, Boston, USA) and luminescence ATP detection was assessed using a MicroBeta Trilux (PerkinElmer). A standard curve was used to determine the amount of ATP as mg of protein. After 24 hours the microtiter plate was taken for scintillation counting of radioactive [¹⁴C]AMG-uptake using a MicroBeta Trilux (PerkinElmer). Subsequently, the mean counts per minute (cpm) were calculated and converted to picomolar (pmol). The obtained radioactive counts were expressed as pmol/mg/hour. Sodium-dependent [14C]AMG-uptake was calculated by subtracting uptake under sodium-free conditions from the uptake obtained in the presence of sodium.

Kinetic parameters, IC₅₀ values and apparent inhibition constant (Ki), were calculated using the Kinetic Enzyme Module (SigmaPlot 8.02, Systat Software, Erkrath, Germany). The inhibitory effect on SGLT transport was also analyzed by IC₅₀ values. Dixon plot analysis was used for both estimation of the inhibition constant (Ki) and for identification of the mechanism of enzyme inhibition. Ki was determined by the point of intersection of two different substrate concentrations. Plots of the reciprocal of rate of metabolite formation (1/Rate) versus inhibitor concentration at each substrate concentration were prepared.

### RESULTS

**Table 1.**

| Thioglucosides tested for inhibitory effect on human SGLT1 and SGLT2. | |
|---|---|
| **Thioglucoside** | **Chemical name** |
| 1 | 8-Hydroxygeranol-1'-thio-β-D-glucopyranoside |
| 2 | 2-Hydroxymethyl-phenyl-1'-thio-β-D-glucopyranoside |
| 3 | Phenyl-1'-thio-β-D-glucopyranoside |
| 4 | *o*-Tolyl-1'-thio-β-D-glucopyranoside |
| 5 | 3-Hydroxymethyl-phenyl-1'-thio-β-D-glucopyranoside |
| 6 | 2-Hydroxymethyl-phenyl-1'-thio-β-D-galactopyranoside |
| 7 | *p*-Tolyl-1'-thio-β-D-glucopyranoside |
| 8 | *m*-Tolyl-1'-thio-β-D-glucopyranoside |

### SGLT Activity

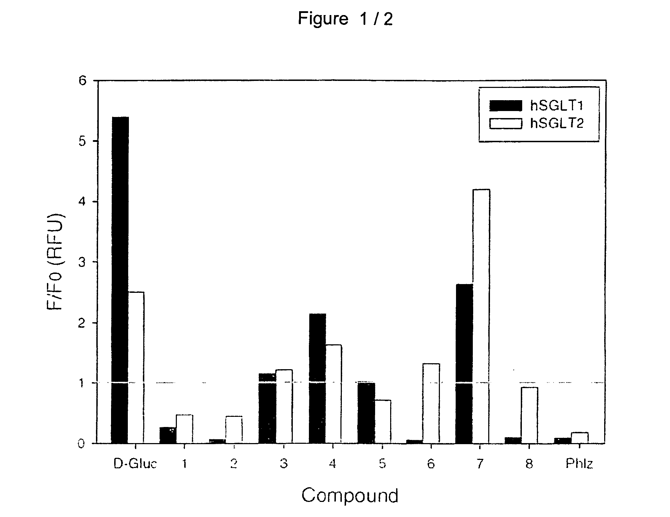

The activity of the sodium-dependent D-glucose cotransport system was studied by changes in membrane potential induced by each of the eight thioglucosides (see Table 1) (10 µm). FRET values were normalized using the fluorescence signal obtained from non-transfected CHO cells. Fluorescence response ratio lower than 1 indicated that such compounds were not significantly transported, while a ratio higher than 1 demonstrated transport across the plasma membrane mediated by SGLT. As shown in Figure 1, hSGLT1 thioglucosides 3, 4, 5, and 7 showed fluorescence signal ratios of 1.15, 2.14. 1.01 and 2.64 (n=16; p<0.01), respectively, while hSGLT2 thioglucosides 3, 4, 6 and 7, had fluorescence ratios significantly higher than one (1.21, 1.63, 1.32 and 4.22, respectively; n=16, p<0.01). These results demonstrate that the thioglucosides 3, 4 and 7 are significantly transported through both hSGLT1 and hSGLT2. Thioglucoside 1, 2 and 8 were not transported by either hSGLT1 or by hSGLT2. The maximal effect was observed with thioglucoside 4 with FRET values higher than 2 for hSGLT1. Thioglucoside 7 had the highest plasma membrane depolarization for hSGLT2 as shown by a FRET value of 4.22. These data clearly demonstrate the presence of electrogenic uptake of some thioglucosides, specifically, thioglucosides 4 and 7, across the plasma membrane. The FRET values of phlorizin demonstrated that this glucose analogue was not transported by hSGLT1 or hSGLT2, thus serving as a control.

### Inhibition of SGLT Transport

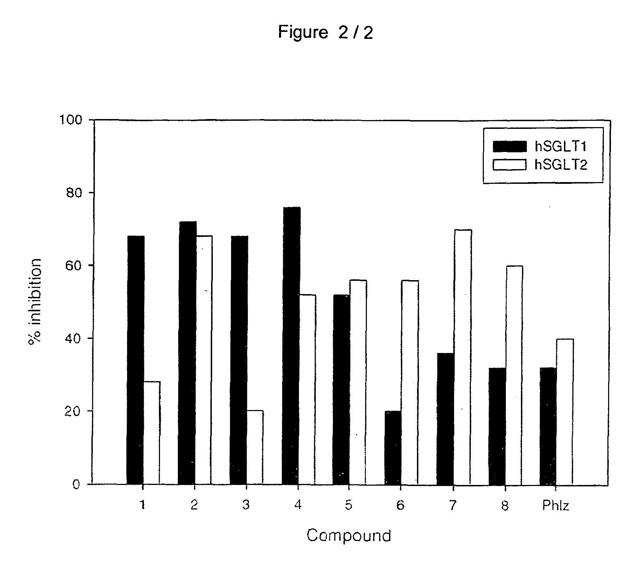

The inhibitory effect of all eight thioglucosides (see Table 1) on sodium-dependent D-glucose co-transport, was evaluated using [¹⁴C]AMG-uptake. Figure 2 shows the uptake values for all thioglucosides and phlorizin (10µM each) expressed as % inhibition. The inhibitory effect of thioglucosides 1, 2, 3 and 4 was stronger for hSGLT1, while thioglucosides 5 through 8 had a higher inhibitory effect for hSGLT2 (n=16, p<0.001). These values were comparable to that obtained with phlorizin (10µM) (n=16), suggesting a strong inhibitory effect for the tested thioglucosides. The inhibitory effect of thioglucosides 4 and 7, both with a maximal transport across the plasma membrane, was above 70% for hSGLT1 and hSGLT2, respectively.

### IC₅₀ and Ki values

To analyze further the inhibitory effect of each thioglucosides 1 through 8, kinetic studies were performed. As shown in Table 2, IC₅₀ values for hSGLT1 were between 30 µM and 120 µM. The only exception was compound 3 with a value of 4 µM, indicating a strong inhibitory effect. The inhibition of hSGLT2 transport was more pronounced as shown by IC₅₀ values between 9 µM and 18 µM. Thioglucoside 4 and 7, however, showed lower inhibitory effect with IC₅₀ values equal to 30 µM and 52 µM for hSGLT1 and 18 µM and 32 µM for hSGLT2, respectively. Thus, thioglucoside 4 and 7 were significantly transported but the inhibitory effect was reduced. However, these values were similar to those obtained with phlorizin with values of 28 µM and 62 µM for hSGLT1 and hSGLT2, respectively.

The apparent inhibition constants (Ki) for all eight thioglucosides are also shown in Table 2. The order of inhibition (lower Ki to higher Ki) of hSGLT1 was compound 3>1 >5>8>4, while of hSGLT2 was 6>2>5>8>1.

**Table 2.**

| IC₅₀ values and apparent inhibitory constant (Ki) of thioglucosides 1 - 8 on hSGLT1 and hSGLT2, values are expressed as µM. | | | | |
|---|---|---|---|---|
| **Thioglucoside** | **hSGLT1** | | **hSGLT2** | |
| | **IC₅₀** | **Ki** | **IC₅₀** | **Ki** |
| 1 | 35 | 0.10 | 12 | 0.03 |
| 2 | 41 | 383 | 9 | 0.01 |
| 3 | 4 | 0.08 | 16 | 135 |
| 4 | 30 | 0.28 | 18 | 132 |
| 5 | 47 | 0.12 | 12 | 0.02 |
| 6 | 120 | 140 | 15 | 0.01 |
| 7 | 52 | 23 | 32 | 175 |
| 8 | 40 | 0.17 | 11 | 0.02 |
| phlorizin | 28 | 15 | 62 | 10 |

Thioglucosides 2 and 6 showed a Ki higher than 100 µM for hSGLT1, while both compounds had a Ki of 0.01 µM for hSGLT2, demonstrating a strong inhibitory effect on hSGLT2 but not on hSGLT1. In addition, the Ki of phlorizin for hSGLT1 and hSGLT2 was 15 µM and 10 µM, respectively, suggesting a stronger inhibitory effect of these thioglucosides as compared to phlorizin. These results demonstrate the significant therapeutic effect of thioglucosides 2 and 6, based on a strong inhibitory effect on hSGLT2 (with Ki = 0.01 µM).

## Claims

1. Compounds having the general formula (I): 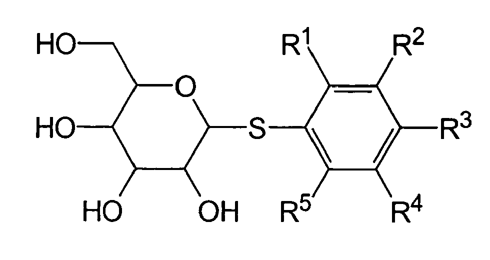 wherein
R¹- R⁵ represent independently of each other
-R⁶, -R⁷, -R⁸, -R⁹, -R¹⁰, -NHR¹¹, -NHR¹², -NR¹³R¹⁴, -NR¹⁵R¹⁶, -OR¹⁷, -OR¹⁸, -COR¹⁹, -COR²⁰, -CONH-R²¹, -CONH-R²², -COOR²³, -COOR²⁴, -OOC-R²⁵, -OOC-R²⁶, -NHCO-R²⁷, -NHCO-R²⁸, -R²⁹ , -R³⁰ , -R³¹ , -R³² , -R³³;
R⁶ - R²⁸ represent independently of each other
-R³⁴, -R³⁵, -R³⁶, -R³⁷, -R³⁸, -CH₂-R³⁹, -C₂H₄-R¹⁰, -C₃H₆-R⁴¹, -C₄H₈-R⁴², -C₅H₁₀-R⁴³, -C₆H₁₂-R⁴⁴, -C₇H₁₄-R⁴⁵, -CHR⁴⁶R⁴⁷, -CR⁴⁸R⁴⁹R⁵⁰, -CHR⁵¹-CHR⁵²R⁵³, -CR⁵⁴R⁵⁴-CR⁵⁶R⁵⁷R⁵⁸, -CHR⁵⁹-CHR⁶⁰-CHR⁶¹R⁶², -CR⁶³R⁶⁴-CR⁶⁵R⁶⁶-CR⁶⁷R⁶⁸R⁶⁹; 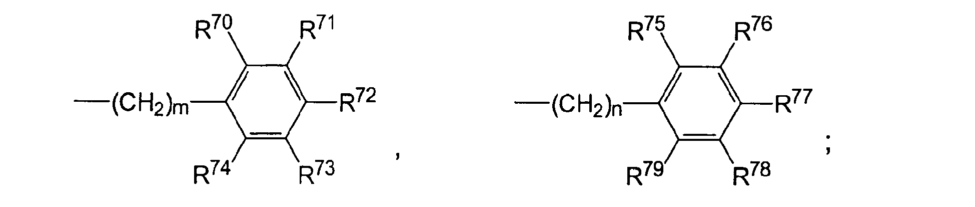
R²⁹- R⁷⁹ represent independently of each other
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -l, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)NH₂, -NH-C(=NH)-NHCH₃, -NH-C(=NH)-NHC₂H₅, -NH-C(=NH)-NHC₃H₇, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂. -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃. -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH2, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -CH₂-CH=CH-CH₂-CH=CH₂, -CH=CH-C₂H₄-CH=CH₂, -C₆H₄-OH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH₂-C₆H₄-OCH₃, -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH,)-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡-C-C≡CH, -CH(C≡CH)-CH₂-C≡CH, -C(C≡CH)₂-CH₃, -CH₂-CH(C≡CH)₂, -CH(C≡CH)-C≡C-CH₃;
m is the integer 1, 2, 3, 4, 5, 6, 7 or 8;
n is the integer 1, 2, 3, 4, 5, 6, 7, 8, 9 or 10;
and wherein at least one substituent R¹ - R⁵ is different from hydrogen,
and stereoisomeric forms, enantiomers, diastereomers, tautomers, anomers, keto-forms, deoxy-forms, prodrugs, solvates, hydrates and pharmaceutically acceptable salts thereof.

2. Compound according to claim 1 having the general formula (II): 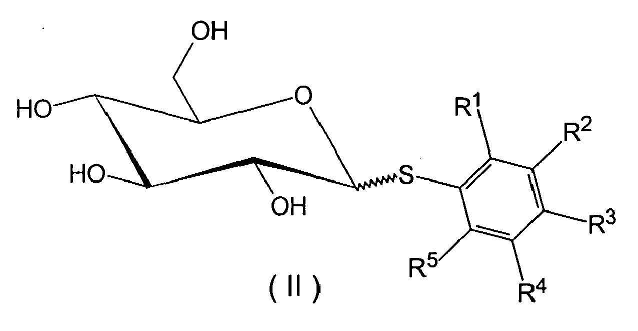 wherein the substituents R¹ - R⁵ have the meanings as disclosed in claim 1.

3. Compound according to claim 1 having the general formula (III): 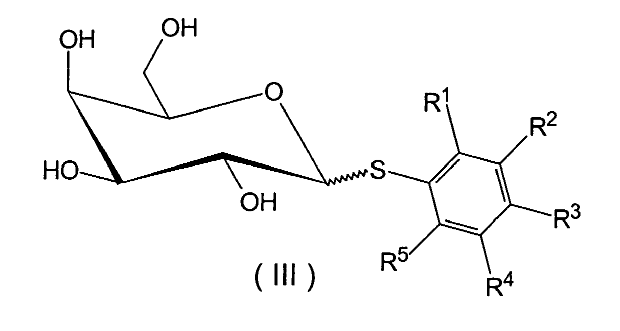 wherein the substituents R¹ - R⁵ have the meanings as disclosed in claim 1.

4. Compound according to any previous claim for use as a pharmaceutically active agent.

5. Use of a compound according to any previous claim for the manufacture of the pharmaceutical composition useful as an inhibitor of a human sodium dependent glucose transporter 2 (SGLT2).

6. Use of a compound according to claim 5 for prophylaxis and/or treatment of diseases and conditions associated with glucose transport by SGLT2.

7. Use of a compound according to claim 5 or 6 for prophylaxis and/or treatment of hyperglycemia and hyperglycemia related diseases and conditions.

8. Use of a compound according to claim 7 wherein the hyperglycemia and hyperglycemia related disease or condition is selected from the group comprising:
type I diabetes; type II diabetes; diabetic complications including coronary heart disease, retinopathy, nephropathy, neuropathy and delayed wound healing; hyperinsulinemia; insulin resistance; obesity; hyperlipidemia including hypertriglyceridemia; metabolic syndrome X, also called insulin resistance syndrome; atherosclerosis; hypertension and related diseases.

9. Use of a compound according to any one of claims 6-7 wherein the hyperglycemia and hyperglycemia related disease or condition is selected from the group comprising:
type I diabetes; type II diabetes; and diabetic complications including coronary heart disease, retinopathy, nephropathy, neuropathy and delayed wound healing.

10. Use of a compound according to any one of claims 1 - 4 in combination with an anti-diabetic or anti-hyperglycemic agent such as insulin and insulin analogues, insulin sensitizers, meglitinides, bigaunides, sulfonyl ureas, PPAR inhibitors, glucagon-like peptide-1 (GLP-1), PTP1B inhibitors, glycogen phosphorylase inhibitors and glucose-6-phosphatase inhibitors or agents for treating diabetic complications such as PKC inhibitors or AGE inhibitors.

11. Use of a compound according to any one of claims 1 - 4 in combination with a lipid-lowering agent such as HMG CoA reductase inhibitors, MTP inhibitors, squalene synthetase inhibitors, fibric acid derivatives, ACAT inhibitors, lipooxygenase inhibitors, cholesterol absorption inhibitors, ileal Na⁺/bile acid cotransporter inhibitors, upregulators of LDL receptor activity, bile acid sequestrants, and nicotinic acid

12. Use of a compound according to any one of claims 1 - 4 in combination with an anti-obesity agent such as beta 3 adrenergic agonist, a lipase inhibitor, a serotonin reuptake inhibitor, and thyroid receptor beta drug.

13. Pharmaceutical composition comprising at least one compound according to any one of claims 1 to 4 as an active ingredient, together with at least one pharmaceutically acceptable carrier, excipient and/or diluents.
